# EUROPEAN PATENT SPECIFICATION

(11) **EP 4 304 485 B1**
(45) Date of publication and mention of the grant of the patent: **12.11.2025**
(21) Application number: 22716471.2
(22) Date of filing: 09.03.2022
(51) Int. Cl.: A61B 8/12, A61B 8/00

(54) **CATHETER WITH ROTATABLE IMAGE ARRAY**
KATHETER MIT DREHBARER BILDANORDNUNG
CATHÉTER DOTÉ D'UN RÉSEAU D'IMAGES ROTATIF

(30) Priority: 09.03.2021 US 202163158604 P
(43) Date of publication of application: 17.01.2024
(73) Proprietor: Oneprojects Design and Innovation Ltd., Dublin, D04 K5D0 (IE)
(72) Inventor: HENNERSPERGER, Christoph, 84453 Muhldord, Bavaria (DE); LAHART, Fionn, Dublin, D6BW KN26 (IE); HENEGHAN, Paul, Dublin, D05R674 (IE); RESTIVO, Courtney, 80634 Munich (DE)
(74) Representative: HGF
(86) International application number: PCT/IB2022/000101
(87) International publication number: WO 2022/189853

(56) References cited:
- US-A1- 2007 167 813
- US-A1- 2008 287 797
- US-A1- 2014 180 128
- US-A1- 2014 257 105
- US-A1- 2014 276 087
- US-B2- 8 398 551

## Description

### Cross-Reference to Related Applications

This application claims priority to, and the benefit of, U.S. Provisional Application No. 63/158,604, filed March 9, 2021.

### Field of the Invention

The invention generally relates to intravascular tissue visualization and characterization. More specifically, the invention relates to an imaging device in the form of a disposable catheter having a uniquely configured imaging assembly, including a rotatable transducer array, provided at a distal portion thereof enabling full circumferential, three-dimensional (3D) ultrasound imaging for providing a 360-degree visualization of intravascular tissue.

### Background

The development of new medical technologies and equipment has provided an increasing number of options available to doctors for the diagnosis and treatment of cardiovascular diseases. For example, ultrasound imaging technologies have enabled doctors to create and view a variety of images generated by one or more sensors inserted within a vasculature.

One ultrasound imaging technology that has been employed is intravascular ultrasound (IVUS). In IVUS imaging systems, an ultrasonic transducer assembly is attached to a distal end of a catheter. The IVUS catheter is carefully maneuvered through a patient's body, usually over a guide wire, to an area of interest such as within a coronary artery. The transducer assembly transmits ultrasound waves and receives echoes from those waves. The received echoes are then converted to electrical signals and transmitted to processing equipment where, for example, an image of the area of interest may be displayed. It should be noted that, for intracardiac echocardiography (ICE) procedures, imaging catheters are similarly configured as IVUS catheters, but are used in larger vessels, and are navigated freely using dedicated deflection/steering mechanisms.

Current imaging catheters utilize a variety of different ultrasound configurations. Some catheters utilize a side firing element or array at the distal end of the catheter, wherein the array is rotated about the longitudinal axis of the catheter. Other catheters include an array of transducer elements arranged circumferentially around the longitudinal axis of the catheter. Yet still, other catheters include a linear array of transducer elements arranged along the longitudinal axis of the catheter body.

While such designs provide some form of ultrasound imaging, these designs suffer from drawbacks. In particular, in typical ultrasound systems configured to visualize inner body regions, dynamic forces are often employed, resulting in a dynamic movement of the body regions over time. These dynamic forces and movements make it difficult to stabilize internal imaging devices and generate consistent and accurate images if imaging of the structure cannot be enabled in real-time (e.g., >20 Hz). As a result, the captured images often lack the necessary quality required to prescribe appropriate treatment or therapy, and internal real-time imaging is limited to small two-dimensional areas or three-dimensional volumetric regions respectively. Furthermore, conventional ultrasound catheters are configured in such a way that tissue and anatomic structures tend to change the spacing or even contact the image acquisition elements making the images difficult to analyze. Yet still, some designs incorporate an air-gap feature, which can be detrimental to the overall imaging capabilities of the catheter as the air-gap is unfavorable, particularly for acoustic coupling purposes. In turn, current imaging catheters may lack the ability to generate consistent and accurate images within the vasculature. US2014257105A1 relates to a fluid fillable catheter capsule assembly is provided suitable for use with an appropriate catheter in imaging applications for medical or surgical procedures. US2008287797A1 relates to imaging catheter tips which contain acoustic transducers for obtaining ultrasound images. US8398551B2 relates to a capacitive ultrasonic transducer, into which a silicon semiconductor substrate is processed using a silicon micromachining technique, and its production method, and to a capacitive ultrasonic probe comprising the capacitive ultrasonic transducer in an end portion of an insertion section inserted into a body cavity. US2007167813A1 relates to ultrasonic probes, and particularly ultrasonic probes for catheter systems and provided in catheter tips for use with catheter systems. US2014276087A1 relates to a method of fabricating miniature ultrasound transducers that includes receiving a wafer on which a plurality of miniature ultrasound transducers is formed. US2014/180128 A1 relates to an ultrasound transducer for use in intravascular ultrasound (IVUS) imaging systems; an IVUS imaging system is provided, including an ultrasound emitter and receiver rotationally disposed within an elongate member; an actuator; and a control system controlling emission of pulses and receiving ultrasound echo data associated with the pulses.

### Summary

The invention is defined in the appended claims. The invention provides a disposable imaging catheter including *inter alia* a uniquely configured imaging assembly provided at a distal portion thereof, generally defining a distal tip of the imaging catheter. The imaging assembly includes a rotatable transducer unit enclosed within an outer sleeve and fully encased within a fluid. **In** particular, the outer sleeve is positioned over the rotatable transducer unit in such a manner that a gap is defined between the transducer unit and an interior surface of the outer sleeve. A volume of fluid is received within and completely fills the gap. The fluid is an acoustic coupling fluid for carrying acoustic signals between the rotatable transducer unit and surrounding fluid and/or tissue, such as intravascular fluid and/or tissue. The outer sleeve includes a sonolucent material, thereby enabling the passage of acoustic signals between the transducer unit and surrounding fluid/tissue while avoiding any rotating components within the vasculature during an imaging procedure. The sonolucent material
allows for the passing of acoustic signals with minimal loss of energy and aberration and/or deflection. Furthermore, the outer sleeve may act as the outermost matching layer, in which the sonolucent material matches impedance of the acoustic signals to be transmitted to the surrounding fluid and/or tissue. Accordingly, such a design allows for subsequent generation of a full circumferential, 360-degree view of the surrounding tissue, while maximizing transmission and minimizing loss of quality or increased artifacts due to maximizing the signal transmission from the imaging assembly and through the sonolucent sleeve, via the acoustic coupling fluid.

The imaging assembly is constructed in a multi-layer fashion. In particular, the rotatable transducer unit, which includes one or more acoustic transducers, further includes a shell member encasing the one or more acoustic transducers within. The shell member is comprised of a material having high mechanical stability and rigidity, providing sufficient protection for the internal transducers and electronic connections, and further provides optimal load balancing for concentricity during rotation thereof. The one or more acoustic transducers are aligned with an imaging window provided on the shell member. The imaging window includes certain dimensions (e.g., a specific thickness, for example, or range of thickness) as well as specific material properties allowing for optimal transmission of acoustic signals by accounting for the acoustic coupling fluid, sonolucent outer sleeve, and surrounding environment (i.e., body fluid, intravascular tissue, etc.). The shell member and associated imaging window are sealed from an external environment to thereby prevent fluid ingress into an interior of the outer shell and prevent subsequent fluid contact with the acoustic transducers.

The transducer unit is positioned within an interior of the outer sleeve, such that a gap is defined between an exterior of the transducer unit (i.e., exterior surface of the shell member) and an interior surface of the outer sleeve. The acoustic coupling fluid is provided within the gap such that the transducer unit is completely encased within the fluid and the transducer unit is sealed within the outer sleeve to prevent fluid ingress and/or egress. In some embodiments, the imaging assembly may be preloaded with the acoustic coupling fluid such that the imaging catheter is ready for immediate use. In other embodiments, the acoustic coupling fluid may be loaded into the imaging assembly on-site and just prior to a procedure.

According to the invention, the imaging catheter includes at least one injection port and at least one purge valve operably associated with the outer sleeve. Accordingly, either at the time of manufacturing or on-site in a procedure room, the injection port allows for filling of the imaging assembly with the acoustic coupling fluid and the purge valve allows for purging of any excess fluid or air bubbles from the assembly during the filling process, thereby ensuring that the gap is air-free (which may otherwise cause distortion and interference with the acoustic signal transmissions).

In some embodiments, both the injection port and purge valve may each be positioned at a specific portion of the outer sleeve. For example, in one embodiment, the injection port may be provided at a proximal portion of the outer sleeve while the purge valve is provided at a distal portion, such as at a distal tip of the outer sleeve. In an alternative embodiment, the injection port may be provided at the distal tip and the purge valve may be provided at a proximal portion. Yet still, in other embodiments, either of the injection port and purge valve may be provided at other portions of the imaging catheter. For example, in some embodiments, either or both of the injection port and purge valve may be provided adjacent to a catheter handle or elongate shaft of the imaging catheter to which the imaging assembly is coupled. Accordingly, a fluid pathway may generally connect the injection port, the purge valve, and the imaging assembly. As such, the fluid pathway may extend from the catheter handle and through the elongate catheter shaft and to the imaging assembly such that fluid can be provided to and from the gap defined within the outer sleeve of the imaging assembly.

The transducer unit is fully rotatable when sealed within the outer sleeve and fully encased within the acoustic coupling fluid, thereby allowing for full circumferential, 360-degree view of the surrounding tissue during a procedure. In particular, a unique connection assembly is provided at an interface between the imaging assembly and a distal portion of the imaging catheter.

According to the invention, the connection assembly includes a torque shaft coupled to a portion of the transducer unit. For example, in one embodiment, a proximal end of the transducer unit is directly coupled to a distal end of a torque shaft extending from the imaging catheter. The torque shaft may include a distal end that includes one or more slots or grooves defined therein and the transducer unit, specifically the shell member, may include one or more corresponding connection members (e.g., in the form of protrusions or the like) shaped and/or sized to be received within the one or more slots or grooves of the distal end of the torque shaft. Upon connection between the distal end of the torque shaft and the shell member of the transducer unit, operation of the torque shaft results in rotational force to be transmitted to the transducer unit, thereby causing corresponding rotation thereof. In some embodiments, one or more alignment rings maybe provided at an interface between the imaging assembly (i.e., the transducer unit and outer sleeve) and the distal portion of the imaging catheter (i.e., the torque shaft and catheter body). The alignment rings ensure smooth and concentric rotation of the transducer unit with the torque shaft while providing a bonding region in which the imaging assembly may be sealed to the distal portion of the imaging catheter.

In some embodiments, the imaging assembly may include one or more bearings, including a first bearing generally provided at a more proximal end of the imaging assembly and a second bearing generally provided at a more distal end of the imaging assembly. In addition to assisting in the rotation of at least the transducer unit, the first and second bearings may also serve as creating the gap within the outer sleeve (i.e., the gap defined between the transducer unit and the interior surface of the outer sleeve) and maintaining a uniform distance to the outer sleeve (whether rotating or not). In such an embodiment, the first and second bearings may be coupled to the shell member of the transducer unit, such that rotation of the bearings results in rotation of the shell member, and thus the acoustic transducers within. Accordingly, in some embodiments, at least the first bearing may be directly coupled to a distal end of the torque shaft.

The torque shaft may comprise a single or multilayer construction. For example, in some embodiments, the torque tube may include a helical stranded cable assembly configured to provide sufficient and stable transmission of torque from the proximal to the distal end thereof, while maintaining sufficient flexibility for navigation through tortuous anatomy associated with a patient's vasculature. In some embodiments, the torque shaft may include a laser-cut hypotube, for example. The torque shaft may be made from a metal material or a polymer, and may further include a reinforced shaft, such as a braided or coiled construction. Yet still, the torque shaft may have different properties based on the different construction design, such as varied pitch and/or cut pattern provides different mechanical properties. It should be further noted that the torque shaft may vary in composition, properties, material and/or diameter along its length.

The torque shaft is further configured to house one or more cable assemblies within, outside of, or embedded within the shaft. The cable assemblies may be configured to communicate with and transmit data between the one or more acoustic transducers and an ultrasound imaging system (i.e., a console, controller, etc.) for subsequent generation of a full circumferential, 360-degree view of surrounding tissue based, at least in part, on signals received from the acoustic transducers. The cable assemblies may further be coupled to additional components integrated into the imaging assembly, such as sensors (e.g., localization and/or tracking sensors) and/or energy delivery elements (e.g., ablation elements). The cable assemblies may be coupled to the acoustic transducers and any additional components (e.g., sensors or energy delivery elements) by way of a rotatable electrical connector.

The distal portion of the imaging catheter may further include a steerable segment to which the imaging assembly is attached. The steerable segment includes one or more pull wires provided within the catheter body and extending along a length thereof from a proximal end (coupled to a control mechanism) to a distal end of the catheter body. Distal ends of the one or more pull wires are coupled to a pull ring member directly coupled to a proximal portion of the imaging assembly. Application of a pulling force upon proximal ends of to the one or more pull wires results in a corresponding movement of the pull ring, thereby resulting in out-of-plane movement of the imaging assembly. The steerable segment may include any number of pull wires so as to provide the desired movement of the imaging assembly. For example, in some embodiments, the steerable segment may include at least a pair of pull wires allowing for bi-directional (single plane) steering.

In some embodiments, in addition to including an imaging assembly, the catheter may further include additional components providing associated capabilities. For example, portions of the catheter may include sensors (e.g., localization and/or tracking sensors) and/or energy delivery elements (e.g., ablation elements). In one embodiment, one or more sensors may be provided at portions of the catheter or imaging assembly that are static (i.e., do not rotate in correspondence with the transducer unit). Yet still, in other embodiments, one or more sensors may be provided on the imaging assembly, specifically provided on a portion of the transducer unit or a part associated therewith, such that the one or more sensors may be dynamic, in that they may rotate upon rotation of the transducer unit. The one or more sensors may include, for example, localization and tracking sensing, temperature sensing, or similar sensing capabilities. Accordingly, the imaging catheter may further include cable connections provided within the catheter body and extending along a length thereof from a proximal end (coupled to control system) to the distally-positioned sensor.

### Brief Description of the Drawings

FIGS. 1A and 1B are diagrammatic illustrations of an ultrasound system for providing intravascular tissue visualization and characterization using an imaging catheter equipped with an imaging assembly according to some embodiments of the present disclosure.
FIG. 2 is a perspective view of an imaging catheter consistent with the present disclosure.
FIG. 3 is an enlarged, perspective view of the imaging assembly provided at a distal portion of the imaging catheter.
FIG. 4 is an enlarged, perspective view (partly in phantom and partly in section) of the imaging assembly consistent with the present disclosure.
FIG. 5 is a perspective view of the transducer unit disassembled from the other components of the imaging assembly.
FIG. 6 is an exploded, perspective view of the transducer unit illustrating the imaging window separated from the shell member.
FIG. 7 is a side, sectional view of the imaging assembly consistent with the present disclosure.
FIG. 8 is a perspective view of a distal portion of the imaging catheter illustrating a portion of the connection assembly for coupling the imaging assembly to the distal portion of the imaging catheter body.
FIG. 9 is a perspective view illustrating coupling of the transducer unit to the torque shaft by way of a torque shaft collet.
FIG. 10 is a perspective view illustrating the outer sleeve fully positioned over the transducer unit and coupled to the distal portion of the imaging catheter by way of the connection assembly.
FIG. 11 is a perspective view, partly in phantom, illustrating the transducer unit fully positioned within the outer sleeve.
FIG. 12 is a side view, partly in phantom, illustrating the imaging assembly operably coupled to the torque shaft and distal portion of the imaging catheter via the connection assembly.
FIG. 13 is a side view, partly in section, illustrating the imaging assembly operably coupled to the torque shaft and distal portion of the imaging catheter via the connection assembly.
FIG. 14 is an enlarged perspective view, partly in section, illustrating the imaging assembly operably coupled to the torque shaft and distal portion of the imaging catheter via the connection assembly.
FIG. 15 is a perspective, sectional view of an outer shaft of the imaging catheter illustrating positioning of the pull wires within a portion thereof.
FIG. 16 is a side, sectional view of one embodiment of an outer shaft of the imaging catheter constructed via a multi-lumen extrusion process.
FIG. 17 is a side, sectional view of another embodiment of an outer shaft of the imaging catheter having a composite braided construction.
FIG. 18 is a perspective view of (partly in phantom) of another embodiment of an imaging assembly consistent with the present disclosure and coupled to a torque shaft of the imaging catheter, illustrating the inclusion of proximal and distal bearing assemblies.
FIG. 19 is an enlarged perspective view (partly in section) of the imaging assembly of FIG. 18, illustrating a transducer unit in greater detail.
FIG. 20 is an enlarged perspective view (partly in section) of a distal portion of the image catheter shaft illustrating the torque shaft in greater detail.
FIGS. 21, 22, 23, and 24 are graphs illustrating imaging window frequency transmission calculations based on the type of imaging window material.
FIG. 25 is a chart provided a summary of the tested materials and the various transmission calculations.
FIG. 26 is a graph illustrating optical window thicknesses for various materials at a specific frequency.
FIGS. 27 and 28 are graphs illustrating calculated critical angles and minimum frequency for a one way transmission, respectively, for various materials.

### Detailed Description

By way of overview, the present invention described herein is directed to an imaging system for use in minimally invasive procedures in the vasculature. The imaging system includes an imaging device in the form of a disposable catheter having a uniquely configured imaging assembly, including a rotatable transducer array, provided at a distal portion thereof and enabling full circumferential, 3D ultrasound imaging for providing a 360-degree visualization of intravascular tissue. The imaging catheter, by way of the imaging assembly, provides highly accurate and reliable real-time visualization and characterization of intravascular tissue.

The imaging device may be particularly useful for real-time monitoring of intravascular tissue to aid in the treatment of problems associated with the vasculature. In particular, the imaging device is useful in the treatment of atrial fibrillation (AF) and other cardiac disorders, as well as endovascular procedures. Accordingly, the imaging device of the present invention may be useful for intracardiac echocardiography (ICE) procedures. AF is an irregular heartbeat caused by electrical signals originating in the atrial chambers of the heart which disrupts the regular rhythm of the beating heart. AF is treated by isolating the origin of these electrical signals and limiting their transmission by ablation of the cells that surround the location of origin and conduct the electrical impulse. The current catheter-based visualization and monitoring systems are only partially effective in identifying complete ablation through the thickness of the tissue (transmural ablation) to prevent re-establishment of conductive paths. As a result, they do not provide a sufficient solution for differentiating partial or temporal electric block from a permanent one. AF affects over 1% of the global population. As the population gets older, the probability of AF increases. Today, the incidence of AF globally is over 33 million and increasing. Of all the treated patients only about 53% get relief after the first ablation procedure and this number can increase to about 80% after multiple procedures. This lack of monitoring and control of the procedure even with the advanced systems for treatment and current monitoring method leave much to be desired to establish effectiveness of the treatment.

The imaging device of the present invention provides a surgeon with high resolution imaging of vasculature, capable of scanning of the vasculature over time to generate and capture data that allows extraction of images of the surfaces, characterization of tissue depth, and specific tissue characteristics such as tissue state or stiffness. Accordingly, the imaging device is useful for monitoring and controlling treatment of problems of the vasculature. For example, during an AF treatment procedure, the imaging device provides advanced ultrasound imaging of the target site, wherein such advanced imaging provides a surgeon with real-time monitoring of the target site, allowing the surgeon to determine completeness of a given procedure, such as ablation of the intended tissue, including a depth of ablation along a desired path. In this manner, a surgeon can correct the procedure in real-time so as to ensure the appropriate treatment is being provided.

It should be noted that, while the following description focuses on the use of the imaging system of the present invention for monitoring of vasculature during an AF treatment procedure, imaging systems of the present invention can be directly employed for ventricular tachycardia, as well as general lesion monitoring, such as in the denervation of renal arteries, as systems can be used and applied in fields outside of cardiac procedures.

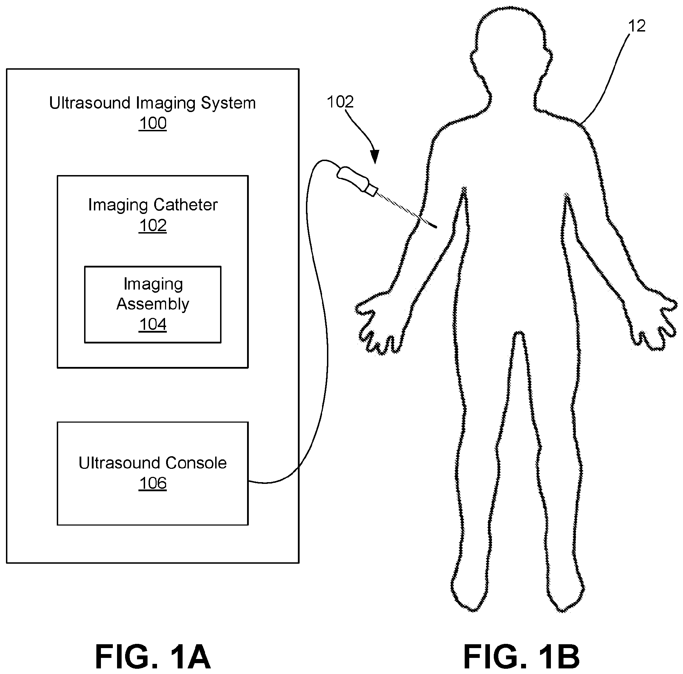

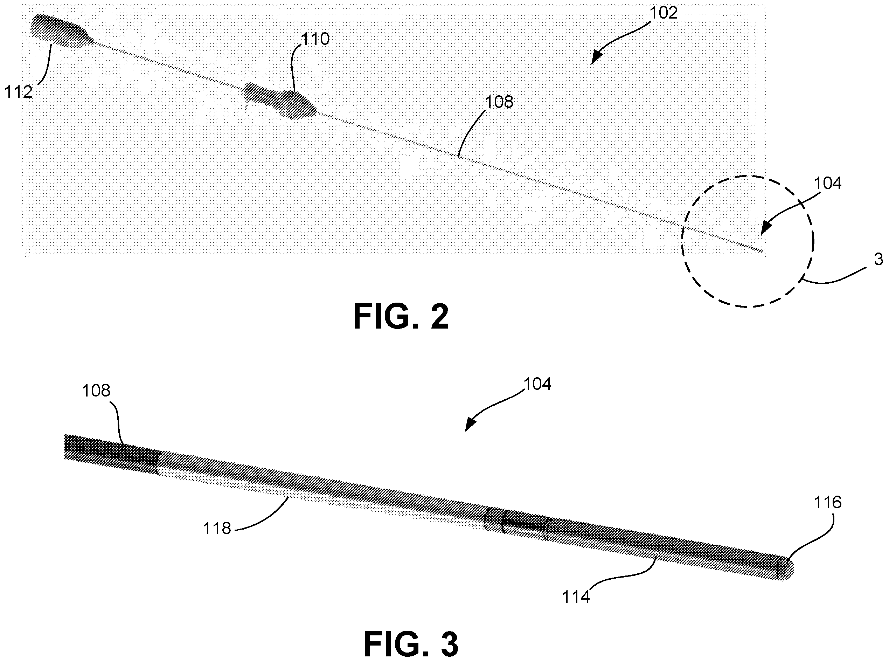

FIGS. 1A and 1B are diagrammatic illustrations of an ultrasound system 100 for providing visualization and characterization of vasculature within a patient 12. The system 100 generally includes an imaging catheter 102 equipped with an imaging assembly 104 and an ultrasound console 106 to which the imaging catheter 102 is to be connected. FIG. 2 is a perspective view of the imaging catheter 102. The catheter 102 includes a catheter body 108, including proximal and distal portions. The imaging assembly 104 is provided at the distal portion, generally defining a distal tip of the imaging catheter. A handle 110 is operably associated with the catheter body 108 and allows for an operator (i.e., surgeon or other medical professional) to manipulate and advance the imaging assembly 104 and the catheter body 108 to a desired target site within the patient's vasculature. The handle 110 may include user-operable inputs for controlling various features and functions of the imaging assembly 104, as will be described in greater detail herein. An interface member 112 is provided at a distal portion of the catheter body 108. The interface member 112 generally provides a connection between the imaging catheter 102, including the imaging assembly 104 and handle 110, and the console 106 for transmission of signals therebetween. The connection may include at least one of a hardwired and wireless connection, for example.

As will be described in greater detail herein, the imaging assembly 104 may generally include a fully rotatable transducer unit comprised of an ultrasound transducer array configured to transmit ultrasound pulses to, and receive echoes of the ultrasound pulses from, surrounding intravascular tissue during a procedure. Such ultrasound transmissions result in a collection of image data which is received by the console 106 and subsequently reconstructed into one or more images providing visualization and characterization of the surrounding intravascular tissue. In particular, the console 106 may utilize image data received from the imaging assembly 104 to reconstruct one or more images providing full circumferential, 360-degree visualization of the intravascular tissue. The console 106 may process the received image data utilizing certain imaging protocols and algorithms for reconstructing images and subsequently outputting, via a display, the reconstructed images (two-, three-, or four-dimensional images) to an operator depicting visualization of the intravascular tissue, In addition to providing reconstruction of images based on received image data from the imaging assembly 104, the console 106 may further provide control over the imaging assembly 104, including control over the emission of ultrasound pulses therefrom (intensity, frequency, duration, etc.) as well as control over the movement of the ultrasound transducer unit (i.e., controlling rotation, including speed and duration of rotation). For example, the console 106 may be equipped with certain hardware and software for providing such image reconstruction and imaging assembly control, as described in International PCT Application No. PCT/IB2019/000963 (Published as WO 2020/044117) to Hennersperger et al.,

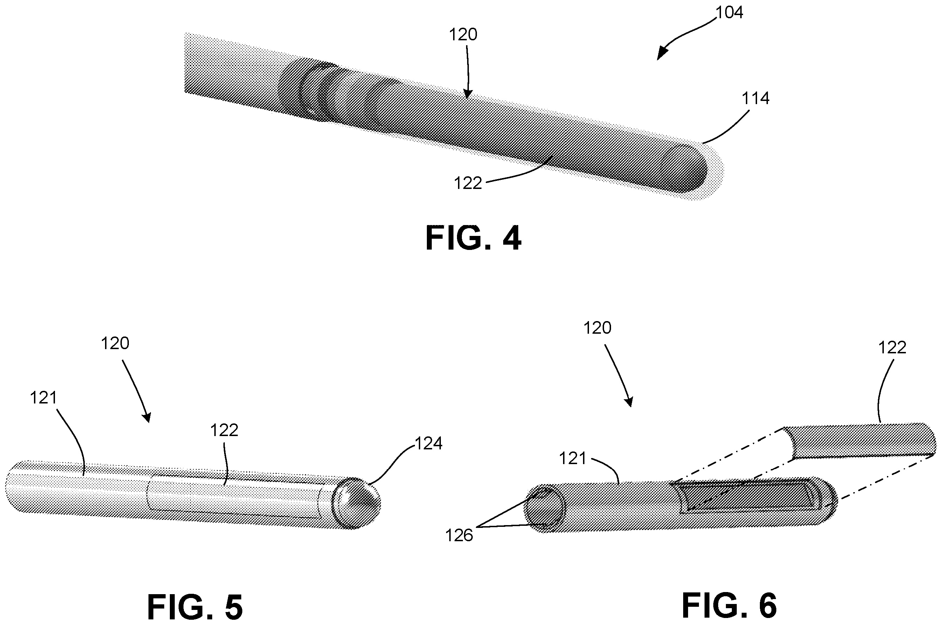

FIG. 3 is an enlarged, perspective view of the imaging assembly 104 provided at a distal portion of the imaging catheter body 108. FIG. 4 is an enlarged, perspective view (partly in phantom and partly in section) of the imaging assembly 104, illustrating internal components in greater detail. The imaging assembly 104 may generally include an imaging portion (i.e., a portion configured for the transmission and receipt of ultrasound pulses to surrounding tissue) and a steerable portion (i.e., steerable segment 118) allowing for movement (e.g., out-of-plane movement) of the imaging portion. The imaging portion includes a fully rotatable transducer unit 120 enclosed within an outer sleeve 114. In particular, the outer sleeve 114 is positioned over the rotatable transducer unit 120 in such a manner that a gap is defined between the transducer unit 120 and an interior surface of the outer sleeve 114. A volume of fluid is received within and completely fills the gap. A distal tip 116 is bonded to a distal portion of the outer sleeve 114 to thereby seal the interior volume the fluid within the interior of the outer sleeve 114 and maintain fluid surrounding the transducer unit 120. The fluid is an acoustic coupling fluid for carrying acoustic signals between the rotatable transducer unit 120 and surrounding tissue. For example, the fluid may include water.

As will be described in greater detail herein, the imaging catheter 102 may include at least one injection port and at least one purge valve operably associated with the imaging assembly 104. Accordingly, either at the time of manufacturing or on-site in a procedure room, an injection port allows for filling of the imaging assembly 104 with the acoustic coupling fluid and the purge valve allows for purging of any excess fluid or air bubbles from the assembly 104 during the filling process, thereby ensuring that the gap defined between the interior of the outer sleeve 114 and transducer unit 120 is air-free (which may otherwise cause distortion and interference with the acoustic signal transmissions).

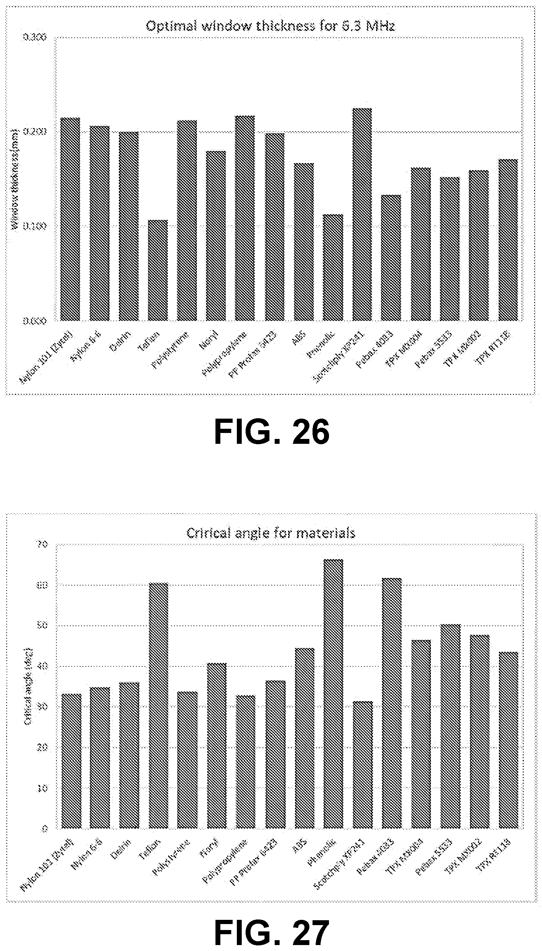

The outer sleeve 114 may include a material exhibiting sonolucent properties (i.e., materials allowing passage of ultrasonic waves without production of echoes that are due to reflection of some of the waves). Such materials may include, but are not limited to, TPX, Pebax, or the like. A full list of materials exhibiting such sonolucent properties are includes in FIGS. 26-28. Accordingly, the outer sleeve 114 enables the passage of ultrasound signals between the transducer unit 120 and surrounding tissue while avoiding any rotating components within the vasculature during an imaging procedure. Such a design allows for subsequent generation of a full circumferential, 360-degree view of the surrounding tissue, while maximizing transmission and minimizing loss of quality or increased artifacts due to maximizing the signal transmission from the transducer unit 120 and through the sonolucent outer sleeve 114, via the acoustic coupling fluid.

The specific dimensions (i.e., thickness) as well as specific material for the outer sleeve 114 are selected based on the desired performance characteristics of the imaging assembly 104. In particular, the outer sleeve 114 may include a certain sonolucent material having a specific thickness so as to achieve an optimal transmission of ultrasound waves, while maintaining of balance of mechanical and acoustic constraints. For example, beam steering performance from the transducer unit 120 is dependent, in part, upon the specific sonolucent material and thickness of such material. Accordingly, an optimal thickness of the outer sleeve 114 may be selected based on the specific sonolucent material to be used and the associated frequency propagating therethrough.

It should be noted that the outer sleeve 114 and distal tip 116 may include certain materials exhibiting specific properties with respect to flexibility and the like. For example, in some embodiments, the outer sleeve 114 may include a first material and the distal tip 116 may include a second material, wherein the first and second materials each exhibit a different hardness (as measured in terms of a durometer). For example, first material (from which the outer sleeve is constructed) may include a relatively high durometer elastomeric material and the second material (from which the distal tip is constructed) may include a relatively low durometer elastomeric material. For example, the outer sleeve 114 may be constructed from a stiffer material while the distal tip 116 may be constructed from more flexible material. By providing the distal tip 116 in a material having a relatively low hardness, the risk of tissue damage during advancement of the imaging assembly 104 within the vasculature can be reduced and/or entirely prevented (i.e., prevention of puncturing the vascular system during advancement of the catheter).

It should be noted that the imaging catheter 102 may not be limited to simply providing imaging and mapping capabilities. Rather, in some embodiments, in addition to including an imaging assembly 104, as described herein, the catheter 102 may further include additional components providing associated capabilities. For example, portions of the catheter may include sensors (e.g., localization and/or tracking sensors) and/or energy delivery elements (e.g., ablation elements). Such components may either be integrated into the imaging assembly 104 itself or provided in associated sections/assemblies of the catheter adjacent to or near the imaging assembly. For example, in some embodiments, the imaging assembly 104 may be included with an active imaging section, located proximal or distal to another active section of the catheter that is used for ablation purposes, electrical mapping purposes, or the like.

In one embodiment, one or more sensors may be provided at portions of the catheter or imaging assembly that are static (i.e., do not rotate in correspondence with the transducer unit). Yet still, in other embodiments, one or more sensors may be provided on the imaging assembly, specifically provided on a portion of the transducer unit or a part associated therewith, such that the one or more sensors may be dynamic, in that they may rotate upon rotation of the transducer unit. The one or more sensors may include, for example, localization and tracking sensing, temperature sensing, or similar sensing capabilities. Accordingly, the imaging catheter may further include cable connections provided within the catheter body and extending along a length thereof from a proximal end (coupled to control system) to the distally-positioned sensor. The one or more sensors may include, for example, localization and tracking sensing, temperature sensing, or similar sensing capabilities. Accordingly, the imaging catheter may further include cable connections provided within the catheter body and extending along a length thereof from a proximal end (coupled to control system) to the distally-positioned sensor.

FIG. 5 is a perspective view of the transducer unit 120 disassembled from the other components of the imaging assembly 104. FIG. 6 is an exploded, perspective view of the transducer unit 120. As illustrated in FIGS. 5 and 6, the rotatable transducer unit 120 includes a shell member 121 encasing the one or more acoustic transducers within. The shell member 121 is comprised of a material having high mechanical stability and rigidity, providing sufficient protection for the internal transducers and any electronic connections, and further provides optimal load balancing for concentricity during rotation thereof. In some embodiments, it may be preferable that the shell member 121 be constructed of a non-metallic material, so as to avoid creation of undesirable artifacts in x-ray data during ultrasound transmission. Accordingly, the material may include polymer material exhibiting high mechanical stability and rigidity. Furthermore, the shell member material may exhibit high electronic isolating properties, so as to provide shielding of internal components, including shielding of an interconnection area in which the acoustic transducers, as well as any additional sensors or components, are coupled to cable assemblies. Yet still, in some embodiments, the shell member 121 may be constructed of a metallic material, such as stainless steel or other medical grade metallic material. A metallic material may be useful in the balancing of the transducer unit 120 so as to provide smooth uniform rotation. Furthermore, a metallic material may provide desired visibility under x-ray imaging during a procedure, such that the shell member 121 essentially provides a visible marker allowing for visualization as to the position of the transducer unit 120 within the vasculature as well as visualization of the rotation thereof. In some embodiments, the shell member 121 may include specific portions (e.g., marker bands or the like) that are intended to be visible under x-ray imaging.

It should be noted that load balancing can also be achieved via other means based on the mechanical design. For example, in some embodiments, it may prove advantageous (from a load-balancing perspective) to remove (e.g., via subtracting manufacturing process) or simply leave out material or add material (e.g., via an additive manufacturing process) around the circumference of the transducer unit so as to mechanically center the mass relative to the catheter axis.

The one or more acoustic transducers are aligned with an imaging window 122 of the shell member 121. The imaging window 122 is provided on at least one side of the shell member 121 extending along a length of the shell member 121 and fixed thereto via an adhesive or the like to thereby provide a sufficient seal to prevent fluid ingress to the interior of the shell member 121. The imaging window 122 includes certain dimensions (e.g., a specific thickness, for example, or range of thickness) as well as specific material properties allowing for optimal transmission of acoustic signals by accounting for the acoustic coupling fluid, sonolucent outer sleeve 114, and surrounding environment (i.e., body fluid, intravascular tissue, etc.). A tip member 124 is fixed to the distal end of the shell member 121 in a similar fashion (i.e., fixed thereto via an adhesive) so as to provide a sufficient seal to prevent fluid ingress to the interior of the shell member 121.

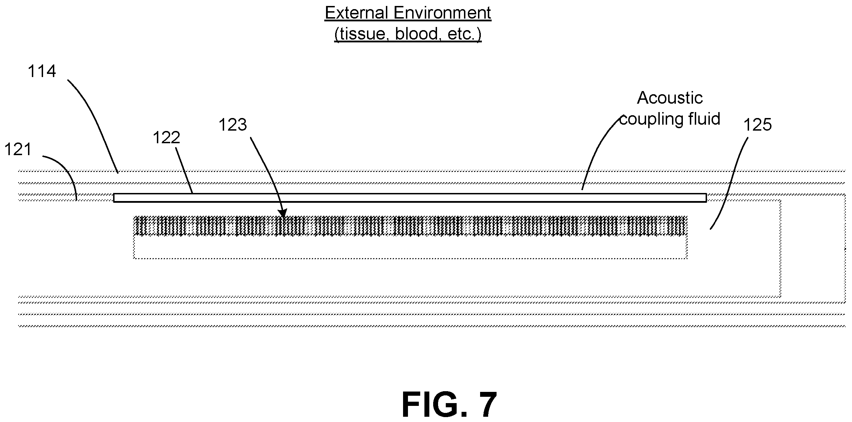

FIG. 7 is a side, sectional view of the imaging assembly 104, illustrating the multi-layer assembly. In particular, the rotatable transducer unit 120 is positioned within the outer sleeve 114. More specifically, the shell member 121, imaging window 122 sealed thereto, and tip member 124 assembly are completely enclosed within the outer sleeve 114. One or more acoustic transducers are housed within the shell member 121, illustrated as an array 123 provided in a Pebax overmold 125. The array 123 is aligned with the imaging window 122. It should be noted that, in addition to providing mechanical housing, the shell member 121 further provides guidance as to the orientation of the of the transducers relative to the imaging window 122. In particular, such alignment can either be manual alignment or the shell member 121 may include a keyed feature to ensure that the transducers are properly aligned concentrically and axially withing the shell member 121 and relative to the imaging window 122.

As shown, the transducer unit is positioned within an interior of the outer sleeve 114, such that a gap is defined between an exterior of the transducer unit (i.e., exterior surface of the shell member) and an interior surface of the outer sleeve. The acoustic coupling fluid is provided within the gap such that the transducer unit is completely encased within the fluid and the transducer unit is sealed within the outer sleeve to prevent fluid ingress and/or egress.

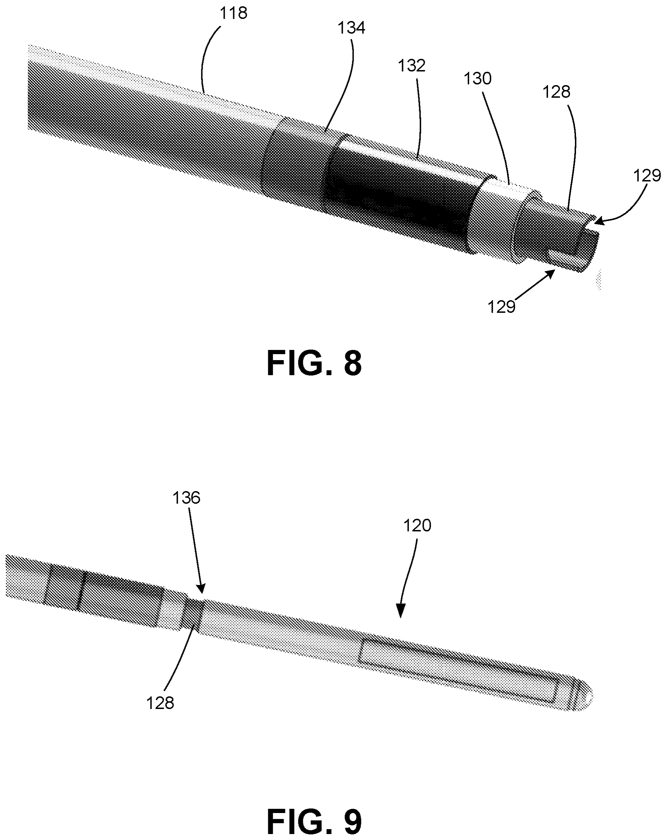

As previously described, the transducer unit 120 may be fully rotatable, thereby allowing for full circumferential, 360-degree view of the surrounding tissue during a procedure. In particular, a unique connection assembly is provided at an interface between the imaging assembly 104, specifically the proximal end of the transducer unit 120, and a distal portion of a torque shaft extending through the imaging catheter body 108. For example, as illustrated in FIG. 6, a proximal end of the shell member 121 includes one or more male connection members 126 (e.g., in the form of protrusions or the like) configured to be coupled to corresponding connection members (e.g., female connection members) associated with a torque shaft. Such an arrangement is described in greater detail herein, particularly with respect to FIGS. 8 and 12-14. Accordingly, upon connection of the proximal end of the shell member 121 to the torque shaft, the transducer unit 120 as a whole is sufficiently sealed from an external environment to thereby prevent fluid ingress into an interior of the shell 121 and prevent subsequent fluid contact with the acoustic transducers.

The one or more transducers may be in the form of an ultrasound transducer array. The array may include a plurality of piezoelectric transducers, typically 1 to 64 or more (e.g. 128, 256, 512, etc.) piezoelectric transducers as transmitters for firing pulses and sensors or receivers for sensing the received echoes. In some embodiments, the ultrasound transducer array includes piezoelectric transducers (e.g., single crystal, composite ceramic, etc.) or alternative transducer designs (e.g., capacitive or piezoelectric micromachined ultrasound transducers, CMUT/PMUT, or other designs) all of which work as both ultrasound transmitters and ultrasound receivers. In some embodiments, the acoustic/ultrasound array comprises transducers configured to operate separately, in which some operate as ultrasound transmitters and other operate as receivers.

The ultrasound transducer array may be configured to function via ultrafast imaging techniques, such as planewave or diverging wave imaging applications. Ultrafast imaging techniques allows for the reconstruction of image data with both superior image quality and high imaging rates. Accordingly, the ultrasound transducer array consistent with the present disclosure may be configured in such a manner so as to allow for ultrafast imaging techniques, as described in International PCT Application No. PCT/IB2019/000963 (Published as WO 2020/044117) to Hennersperger et a .

The transducer unit 120 is fully rotatable when sealed within the outer sleeve and fully encased within the acoustic coupling fluid, thereby allowing for full circumferential, 360-degree view of the surrounding tissue during a procedure. In particular, a unique connection assembly is provided at an interface between the imaging assembly 104 and a distal portion of the imaging catheter body 108.

FIG. 8 is a perspective view of a distal portion of the imaging catheter body 108, extending from the steerable segment 118 portion, and illustrating a portion of the connection assembly for coupling the imaging assembly 104 to the catheter body 108. As shown, the unique connection assembly includes a proximal end of the transducer unit that is directly coupled to a distal end of a torque shaft. According to the invention, the torque shaft includes a collet 128 connected to a distal end thereof. The collet 128 includes one or more female connection members 129 (e.g., in the form of slots or grooves, or other configurations) shaped and/or sized to receive the corresponding male connection members 126 (e.g., the protrusions) provided at the proximal end of the shell member 121. FIG. 9 is a perspective view illustrating coupling of the transducer unit 120 to the torque shaft (not shown) by way of the collet 128 connected to the proximal end of the shell member 121, illustrated at arrow 136. The male and female connection members 126, 129 of the shell member 121 and collet 128, respectively, interlock with one another so as to provide sufficient engagement for the transfer of rotational force from the torque shaft to the shell member 121, thereby resulting in corresponding rotation of the transducer unit 120 upon rotation of the torque shaft.

Upon connection between the distal end of the torque shaft (via collet 128) and the shell member 121 of the transducer unit 120, operation of the torque shaft results in rotational force to be transmitted to the transducer unit, thereby causing corresponding rotation thereof.

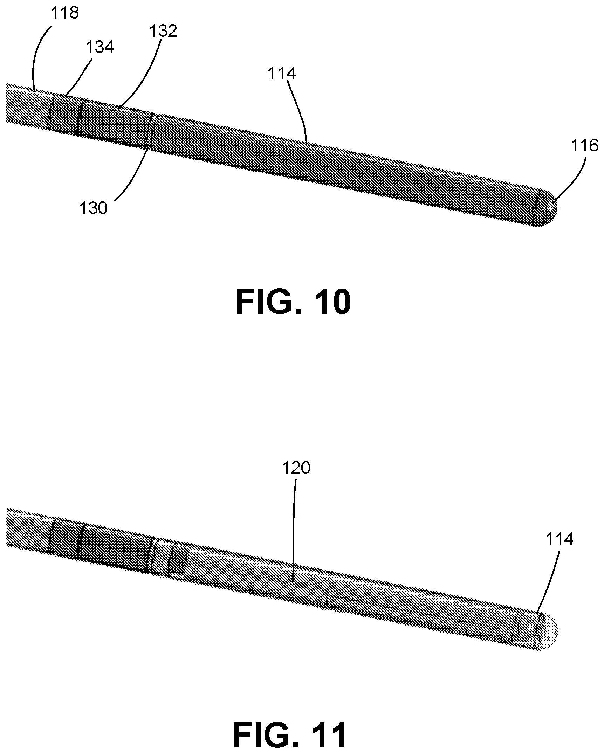

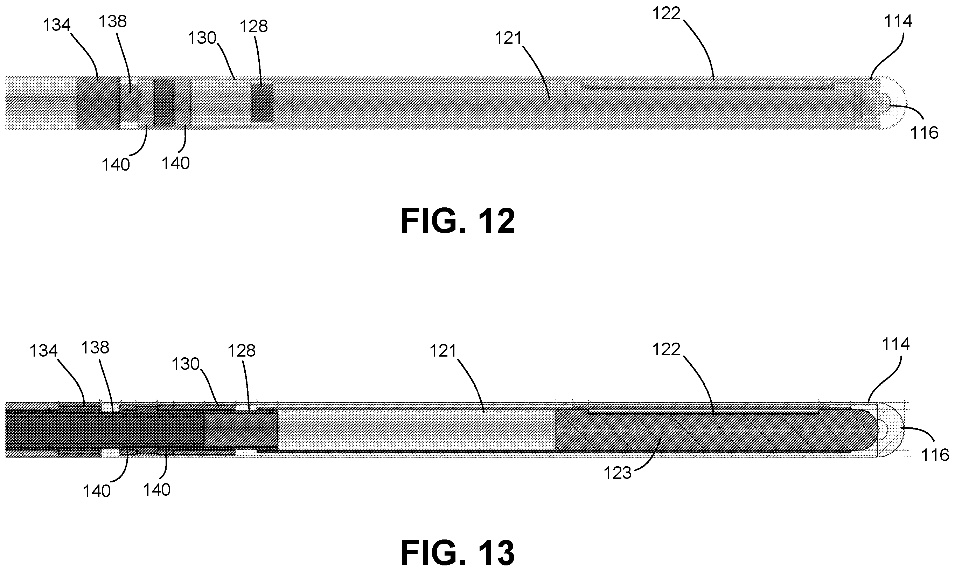

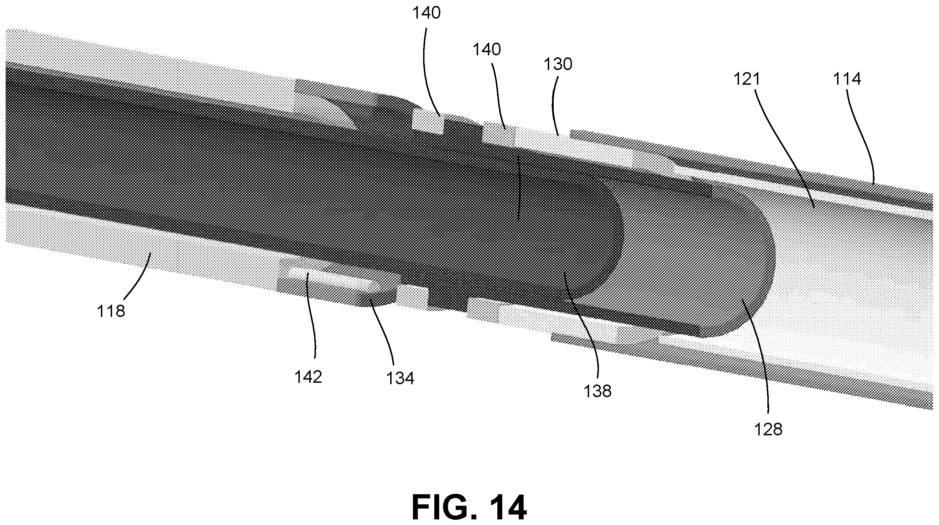

FIGS. 10, 11, 12, 13, and 14 provide additional views illustrating coupling of the transducer unit 120 and outer sleeve 114 to the distal portion of the imaging catheter body 108 by way of the connection assembly. In particular, FIG. 10 is a perspective view illustrating the outer sleeve 114 fully positioned over the transducer unit and coupled to the distal portion of the imaging catheter body 108 by way of the connection assembly. FIG. 11 is a perspective view, partly in phantom, illustrating the transducer unit 120 fully positioned and sealed within the outer sleeve 114. FIG. 12 is a side view, partly in phantom, FIG. 13 is a side view, partly in section, and FIG. 14 is an enlarged perspective view, partly in section, illustrating the transducer unit 120 operably coupled to the torque shaft 138 and the outer sleeve 114 coupled to the distal portion of the imaging catheter body 108 via the connection assembly.

As illustrated, the outer sleeve 114 completely encloses the transducer unit 120 within and is coupled to the distal portion of the catheter body 108 via a sealed engagement between a proximal end of the outer sleeve 114 and a distal collet 130 member, which is positioned over the torque shaft collet 128. The assembly further includes one or more alignment rings 140 provided at the interface between the imaging assembly 104 and the distal portion of the imaging catheter body 108. The alignment rings 140 ensure smooth and concentric rotation of the transducer unit 120 with the torque shaft 138 while providing a bonding region in which the imaging assembly 104 may be sealed to the distal portion of the imaging catheter body 108. A bridging sleeve 132 may be provided over the alignment rings 140. It should be noted that additional alignment rings may be provided at a distal end of the imaging assembly 104.

As shown, the transducer unit 120 is positioned within an interior of the outer sleeve 114, such that a gap is defined between an exterior of the transducer unit 120 (i.e., exterior surface of the shell member 121) and an interior surface of the outer sleeve 114. The acoustic coupling fluid is provided within the gap such that the transducer unit 120 is completely encased within the fluid and the transducer unit 120 is sealed within the outer sleeve 114 to prevent fluid ingress and/or egress. In some embodiments, the imaging assembly 104 may be preloaded with the acoustic coupling fluid such that the imaging catheter 102 is ready for immediate use. In other embodiments, the acoustic coupling fluid may be loaded into the imaging assembly 104 on-site and just prior to a procedure. More specifically, the imaging assembly 104 may further include an injection port (not shown) and a purge valve (not shown) operably associated with the outer sleeve 114.

In some embodiments, both the injection port and purge valve may each be positioned at a specific portion of the outer sleeve. For example, in one embodiment, the injection port may be provided at a proximal portion of the outer sleeve while the purge valve is provided at a distal portion, such as at a distal tip of the outer sleeve. In an alternative embodiment, the injection port may be provided at the distal tip and the purge valve may be provided at a proximal portion. Yet still, in other embodiments, either of the injection port and purge valve may be provided at other portions of the imaging catheter. For example, in some embodiments, either or both of the injection port and purge valve may be provided adjacent to a catheter handle or elongate shaft of the imaging catheter to which the imaging assembly is coupled. Accordingly, a fluid pathway may generally connect the injection port, the purge valve, and the imaging assembly. As such, the fluid pathway may extend from the catheter handle and through the elongate catheter shaft and to the imaging assembly such that fluid can be provided to and from the gap defined within the outer sleeve of the imaging assembly.

Accordingly, either at the time of manufacturing or on-site in a procedure room, the injection port allows for filling of the imaging assembly 104 with the acoustic coupling fluid and the purge valve allows for purging of any excess fluid or air bubbles from the assembly during the filling process, thereby ensuring that the gap is air-free (which may otherwise cause distortion and interference with the acoustic signal transmissions).

It should be noted that, in some embodiments, the injection port and/or purge valve may simply be self-sealing apertures of sorts, each of which may be comprised of silicone or other soft material configured to provide self-sealing functionality. In some embodiments, either of the injection port and purge valve may simply include an aperture that is manually plugged after use (i.e., plugged via a press-fit, screwed, or other mechanical means of closure).

Accordingly, either at the time of manufacturing or on-site in a procedure room, the injection port allows for filling of the imaging assembly with the acoustic coupling fluid and the purge valve allows for purging of any excess fluid or air bubbles from the assembly during the filling process, thereby ensuring that the gap is air-free (which may otherwise cause distortion and interference with the acoustic signal transmissions).

As previously described, the acoustic coupling fluid is a fluid for carrying acoustic signals between the rotatable transducer unit and surrounding fluid and/or tissue, such as intravascular fluid and/or tissue. As such, the fluid may include, for example, water. However, it should be noted that the fluid may include other materials capable of carrying acoustic signals in a desired manner, as well as provide other benefits. For example, the fluid may be used to provide lubrication, such as an oil or the like. The fluid may also provide isolation / dielectric properties which are favorable for EMI purposes. The fluid may also provide acoustic properties allowing the fluid, in combination with the other materials, to form a set of matching layers to tune the acoustic signals to the impedance of the surrounding environment (intravascular tissue and/or fluid).

The torque shaft 138 may comprise a single or multilayer construction. For example, in some embodiments, the torque shaft tube 138 may include a helical stranded assembly configured to provide sufficient and stable transmission of torque from the proximal to the distal end thereof, while maintaining sufficient flexibility for navigation through tortuous anatomy associated with a patient's vasculature. It should be noted that other forms of torque shaft tubes may be included, such as, for example, laser-cut hypotubes and the like. It should be noted that the torque shaft may vary in composition (helical stranded cable/laser cut hypotube etc), properties (pitch/cut pattern, etc.), material, and/or dimensions (i.e., diameter along its length) to achieve the desired effects.

The torque shaft 138 is further configured to house one or more cable assemblies within, outside of, or embedded within. The cable assemblies may be configured to communicate with and transmit data between the one or more acoustic transducers and an ultrasound imaging system (i.e., a console, controller, etc.) for subsequent generation of a full circumferential, 360-degree view of surrounding tissue based, at least in part, on signals received from the acoustic transducers. The cable assemblies may further be coupled to additional components integrated into the imaging assembly, such as sensors (e.g., localization and/or tracking sensors) and/or energy delivery elements (e.g., ablation elements). The cable assemblies may be coupled to the acoustic transducers and any additional components (e.g., sensors or energy delivery elements) by way of a rotatable electrical connector.

The torque shaft 138 may further include an exterior or interior liner so as to further prevent ingress of fluid into an interior thereof and prevent liquid contact with cable assemblies housed within. The exterior liner may further reduce friction between the torque shaft 138 and the surrounding catheter body 108, such that the catheter body 108 remains stationary with respect to rotation of the torque shaft 138.

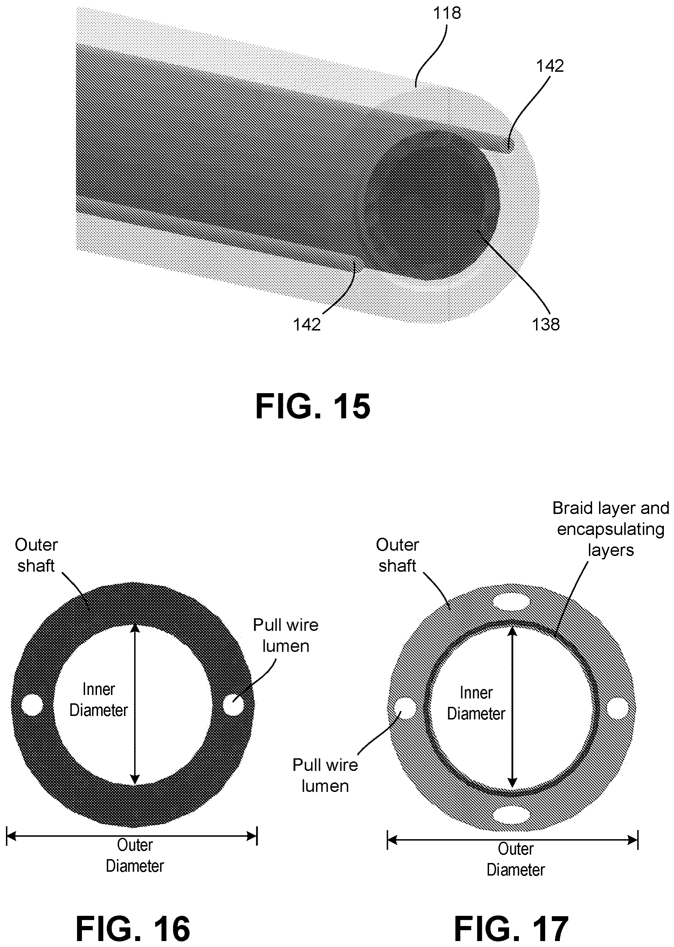

As previously described, the distal portion of the imaging catheter body 108 may further include a steerable segment 118 to which the imaging assembly 104 is attached. As shown in FIG. 14, the steerable segment 118 includes one or more pull wires 142 provided within the catheter body 108 and extending along a length thereof from a proximal end (coupled to a control mechanism) to a distal end of the catheter body 108. FIG. 15 is a perspective, sectional view of the catheter body 108 illustrating positioning of the pull wires within a portion thereof.

Distal ends of the one or more pull wires 142 are coupled to a pull ring member 134 directly coupled to a proximal portion of the imaging assembly 104. Application of a pulling force upon proximal ends of to the one or more pull wires 142 results in a corresponding movement of the pull ring 134, thereby resulting in out-of-plane movement of the imaging assembly 104. For example, the handle member 110 may include certain input mechanisms (i.e., trigger(s), knob(s), lever(s), or the like) that, when actuated by an operator, result in retraction of a given pull wire 142 and associated pull ring 134, and causing corresponding movement (i.e., steering) of the image assembly 104. The imaging catheter 102 may include any number of pull wires so as to provide the desired movement of the imaging assembly. For example, in some embodiments, at least a pair of pull wires may be provided, allowing for bi-directional (single plane) steering. In other embodiments, more than two pull wires may be provided so as to allow for desired out-of-plane movement to occur.

FIG. 16 is a side, sectional view of one embodiment of a catheter body (i.e., outer shaft) of the imaging catheter constructed via a multi-lumen extrusion process. FIG. 17 is a side, sectional view of another embodiment of an outer shaft of the imaging catheter having a composite braided construction. In either construction, the outer diameter of the outer shaft is approximately 0.131 inches which the internal diameter is approximately 0.090 inches. However, it should be noted that, depending on the specific application and intended vasculature in which the imaging catheter 102 will be used, the outer and inner diameters of the outer shaft may vary, as generally understood.

As previously described, the imaging window 122 of the transducer unit 120 includes certain dimensions (e.g., a specific thickness, for example, or range of thickness) as well as specific material properties allowing for optimal transmission of acoustic signals by accounting for the acoustic coupling fluid, sonolucent outer sleeve, and surrounding environment (i.e., body fluid, intravascular tissue, etc.). In order to determine optimal window thickness for a given procedure (i.e., as for a required frequency of ultrasound transmission) and optimal window material, calculations were performed on various materials having the potential to serve as the imaging window material.

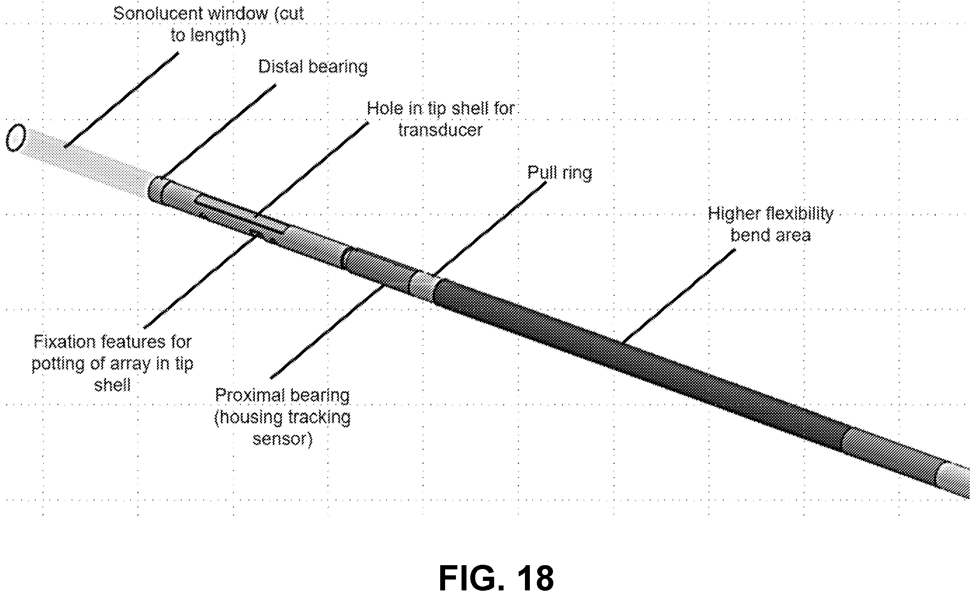

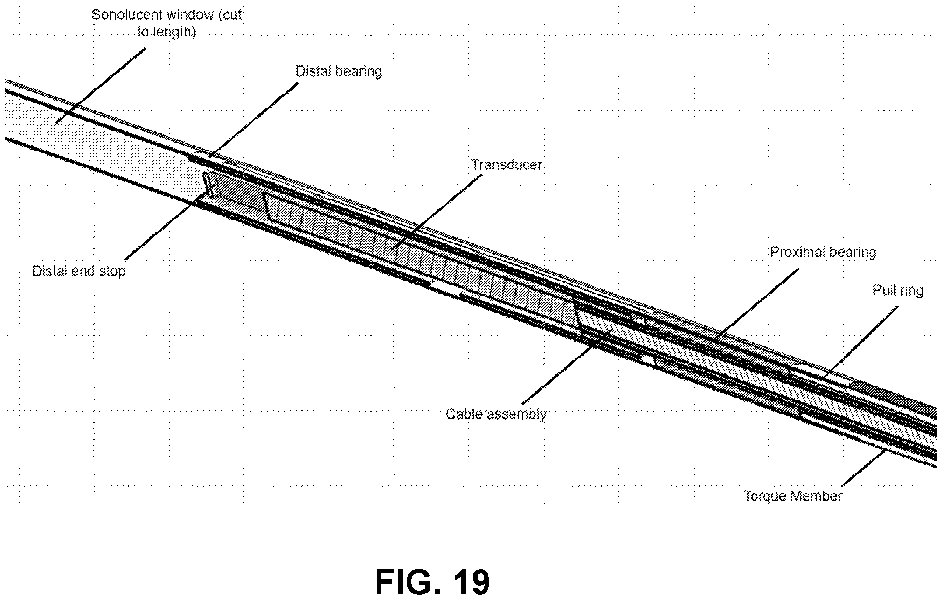

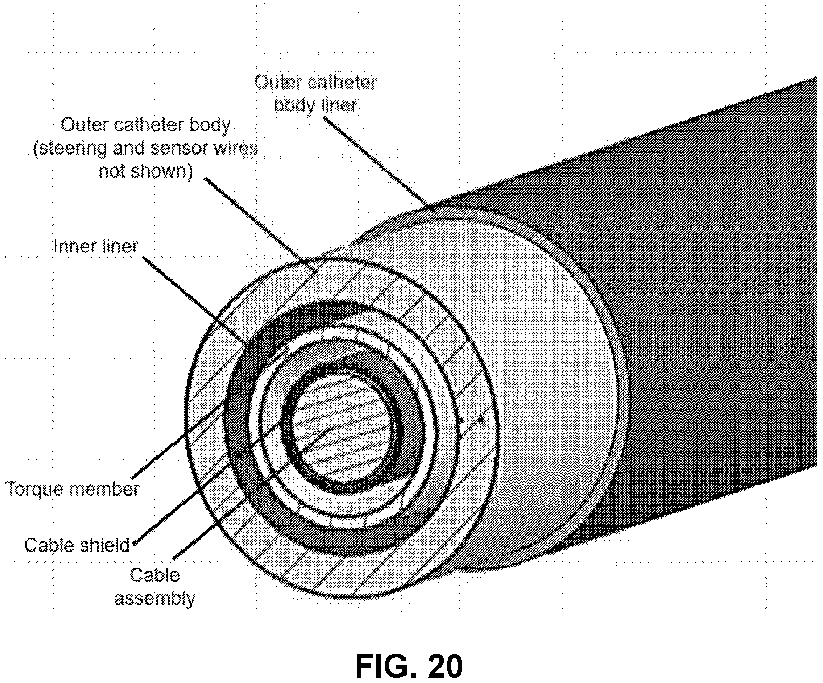

FIGS. 18 and 19 are perspective views of, partly in phantom and partly in section, of another embodiment of an imaging assembly consistent with the present disclosure and coupled to a torque shaft of the imaging catheter. As shown, the imaging assembly includes multiple bearings, including a first bearing generally provided at a more proximal end of the imaging assembly and a second bearing generally provided at a more distal end of the imaging assembly. In addition to assisting in the movement of at least the transducer unit and enabling smooth rotation thereof, the first and second bearings may also serve as creating the gap within the outer sleeve (i.e., the gap defined between the transducer unit and the interior surface of the outer sleeve) and maintaining a uniform distance to the outer sleeve (whether rotating or not). In such an embodiment, the first and second bearings may be coupled to the shell member of the transducer unit, such that rotation of the bearings results in rotation of the shell member, and thus the acoustic transducers within. Accordingly, in some embodiments, at least the first bearing may be directly coupled to a distal end of the torque shaft. The first and second bearings further maintain the transducer unit, specification the acoustic transducer array, concentric within the outer sleeve. FIG. 20 is an enlarged perspective view (partly in section) of a distal portion of the image catheter shaft illustrating the torque shaft in greater detail.

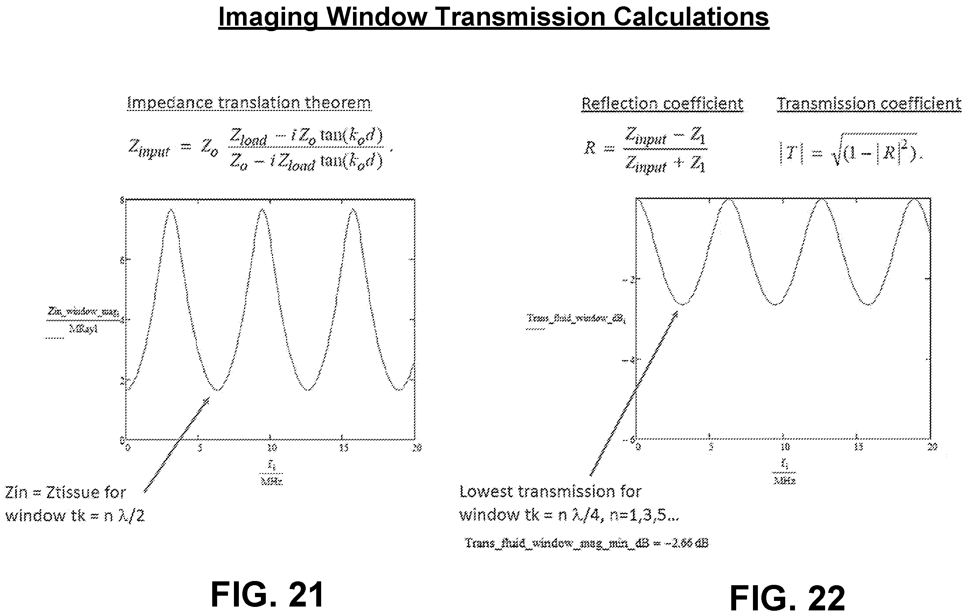

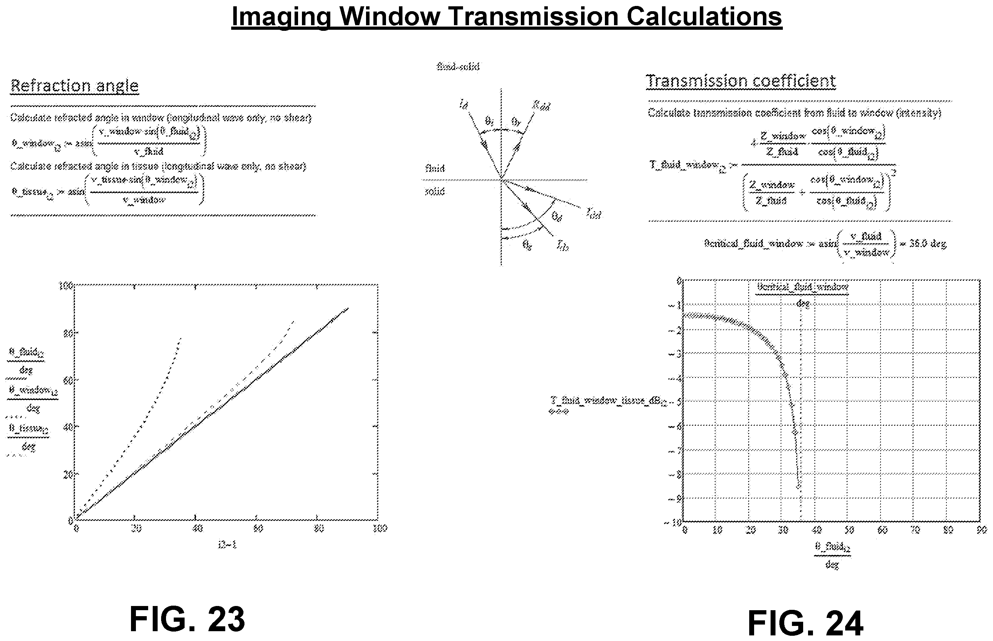

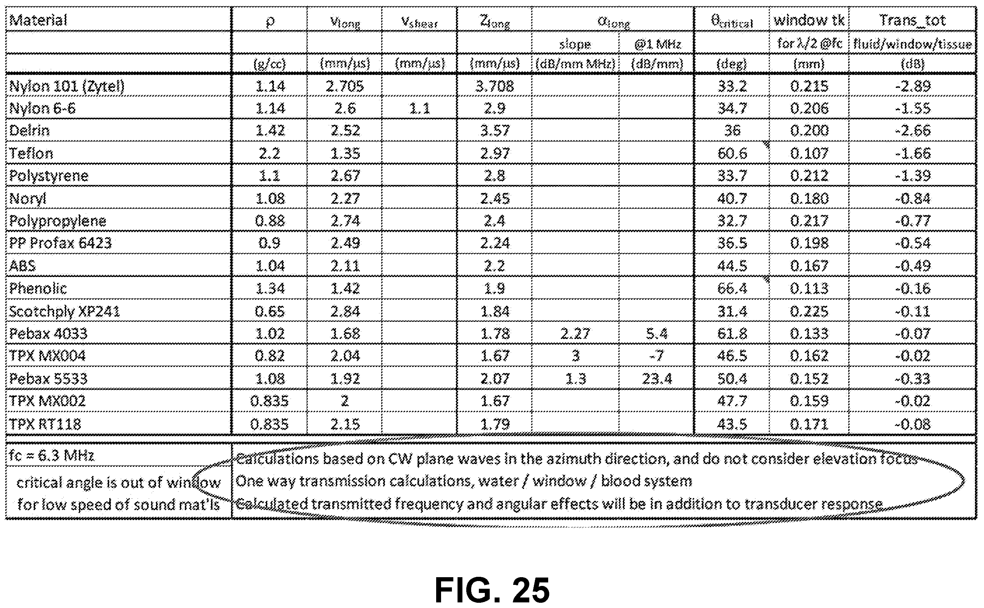

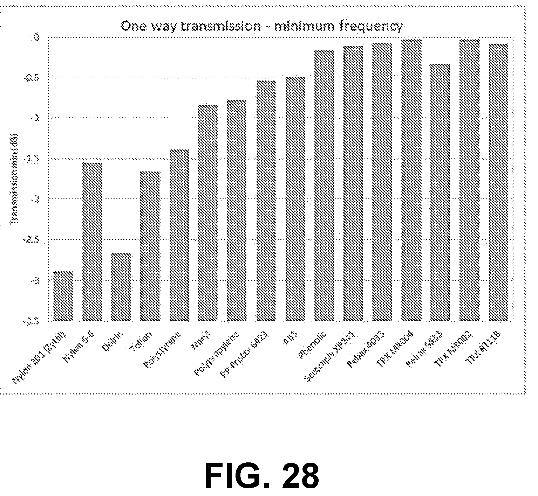

FIGS. 21, 22, 23, and 24 are graphs illustrating imaging window frequency transmission calculations based on the type of imaging window material. FIG. 25 is a chart provided a summary of the tested materials and the various transmission calculations. FIG. 26 is a graph illustrating optical window thicknesses for various materials at a specific frequency. FIGS. 27 and 28 are graphs illustrating calculated critical angles and minimum frequency for a one way transmission, respectively, for various materials.

More specifically, the transmission of wave energy through a material is impacted by the thickness of the material proportional to multiples of half the wavelength for tissue coupling, and multiples of quarter wavelength for fluid coupling reaching minimal values for window thickness. Furthermore the critical angle as maximal usable angle for planewave/diverging wave tilts or beam steering respectively is impacted by the acoustic propagation speed in both the acoustic window and surrounding tissue. A combination of window thickness and critical angle can be used to find the best material-thickness combination acoustically, which is then combined with selection of favorable mechanical properties (mechanical stability, machinability, etc.).

### Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The terms and expressions which have been employed herein are used as terms of description and not of limitation, and there is no intention, in the use of such terms and expressions, of excluding any equivalents of the features shown and described (or portions thereof), and it is recognized that various modifications are possible within the scope of the claims. Accordingly, the claims are intended to cover all such equivalents.

## Claims

1. An imaging catheter (102) comprising:
an imaging assembly (104) provided at a distal portion of the catheter (102), the imaging assembly comprising a fully rotatable transducer unit (120) comprising one or more acoustic transducers encased within a shell (121), wherein the transducer unit (120) is operably associated with a torque shaft (138) configured to cause rotation of the transducer unit (120) upon operation thereof, an outer sleeve (114) positioned over the imaging assembly that encloses the imaging assembly in a manner that a gap is defined between the imaging assembly (104) and an interior surface of the outer sleeve (114) such that a volume of fluid is receivable within the gap, the fluid being an acoustic coupling fluid for carrying acoustic signals between the transducer unit and surrounding tissue, the
imaging catheter **characterised by**, the transducer unit (120) being operably associated with the torque shaft via direct engagement between one or more female connection members (129) of a collet (128) provided at a distal end of the torque shaft and one or more corresponding male connection members (126) provided at a proximal end of the shell (121);
the outer sleeve comprising a sonolucent material matching impedance of acoustic signals to be transmitted to surrounding tissue; and
at least one injection port and at least one purge valve operably associated with the outer sleeve, wherein the injection port is configured for introduction of the volume of fluid into the gap and the purge valve is configured to allow at least a volume of received fluid to be purged from the gap.

2. The imaging catheter of claim 1, wherein one of the injection port and purge valve is provided at a proximal position relative to the imaging assembly (104) and the other is provided at a distal position relative to the imaging assembly (104).

3. The imaging catheter of claim 1, wherein the volume of fluid completely fills the gap.

4. The imaging catheter of claim 1, wherein the acoustic coupling fluid allows for subsequent generation of a full circumferential, 360-degree view of the surrounding tissue based, at least in part, on the acoustic signals.

5. The imaging catheter of claim 1, wherein the shell (121) includes a material comprising high mechanical stability and is sealed from an external environment to thereby prevent fluid ingress to an interior of the shell and into contact with the one or more acoustic transducers.

6. The imaging catheter of claim 1, wherein the one or more acoustic transducers are aligned with an imaging window of the shell.

7. The imaging catheter of claim 1, wherein the outer sleeve (114) comprises one or more portions along a length thereof and each portion comprising a material exhibiting different mechanical properties.

8. The imaging catheter of claim 7, wherein a main body of the outer sleeve (114) comprises a first material and a distal tip member defining a distal tip of the outer sleeve (114) comprises a second material, wherein the first material comprises a relatively high durometer elastomeric material and the second material comprises a relatively low durometer elastomeric material.

9. The imaging catheter of claim 1, further comprising a distal tip (116) bonded to a distal portion of the outer sleeve (114) to thereby seal a fluid within the interior outer sleeve (114).

10. The imaging catheter of claim 9, wherein the distal tip (116) comprises at least one of a purge valve and an injection port, wherein operation of at least one thereof facilitates removal of at least air from the interior of the outer sleeve (114).

11. The imaging catheter of claim 9, wherein the outer sleeve (114) comprises a first material and the distal tip (116) comprises a second material, wherein the first material comprises a relatively high durometer elastomeric material and the second material comprises a relatively low durometer elastomeric material.

12. The imaging catheter of claim 1, further comprising one or more connection members to which at least a proximal end of the outer sleeve (114) is bonded to thereby seal the fluid within the interior of the outer sleeve (114).

13. The imaging catheter of claim 1, wherein the torque shaft (138) comprises a flexible tube for transmitting torque forces upon the transducer unit (120) to cause rotation thereof.

14. The imaging catheter of claim 1, further comprising a cable assembly including one or more wires (142) for carrying signals to and from the transducer unit (120) for subsequent generation of a full circumferential, 360-degree view of surrounding tissue based, at least in part, on the signals.

15. The imaging catheter of claim 14, wherein the cable assembly comprises at least one of a twisted pair of wires, a coaxial cable, and a flexible printed circuit cable, wherein each of which comprises a proximal interconnect portion of wires and a distal interconnect portion of wires.

## Patentansprüche

1. Bildgebungskatheter (102), umfassend:
eine Bildgebungsanordnung (104), die an einem distalen Abschnitt des Katheters (102) bereitgestellt ist, wobei die Bildgebungsanordnung eine vollständig drehbare Wandlereinheit (120) umfasst, die einen oder mehrere akustische Wandler umfasst, die in einer Hülle (121) eingeschlossen sind, wobei die Wandlereinheit (120) betriebsmäßig mit einer Drehmomentwelle (138) verbunden ist, die dazu konfiguriert ist, eine Drehung der Wandlereinheit (120) beim Betrieb davon zu bewirken, eine Außenhülse (114), die über der Bildgebungsanordnung positioniert ist und die Bildgebungsanordnung in einer Weise umschließt, dass ein Spalt zwischen der Bildgebungsanordnung (104) und einer Innenfläche der Außenhülse (114) definiert ist, sodass ein Fluidvolumen innerhalb des Spalts aufnehmbar ist, wobei das Fluid ein akustisches Kopplungsfluid zum Führen akustischer Signale zwischen der Wandlereinheit und umgebendem Gewebe ist, wobei der Bildgebungskatheter **dadurch gekennzeichnet ist, dass**
die Wandlereinheit (120) über einen direkten Eingriff zwischen einem oder mehreren Buchsenverbindungselementen (129) einer Klemmhülse (128), die an einem distalen Ende der Drehmomentwelle bereitgestellt ist, und einem oder mehreren entsprechenden Steckerverbindungselementen (126), die an einem proximalen Ende der Hülle (121) bereitgestellt sind, mit der Drehmomentwelle betriebsmäßig verbunden ist;
wobei die Außenhülse ein sonoluzentes Material umfasst, das
zu der Impedanz von akustischen Signalen, die an umgebendes Gewebe zu übertragen sind, passt;
und zumindest einen Injektionsanschluss und zumindest ein Spülventil, das betriebsmäßig mit der Außenhülse verbunden ist, wobei der Injektionsanschluss zum Einführen des Fluidvolumens in den Spalt konfiguriert ist und das Spülventil dazu konfiguriert ist, zu ermöglichen, dass zumindest ein Volumen des aufgenommenen Fluids aus dem Spalt gespült wird.

2. Bildgebungskatheter nach Anspruch 1, wobei eines von dem Injektionsanschluss und dem Spülventil an einer proximalen Position relativ zu der Bildgebungsanordnung (104) bereitgestellt ist und das andere an einer distalen Position relativ zu der Bildgebungsanordnung (104) bereitgestellt ist.

3. Bildgebungskatheter nach Anspruch 1, wobei das Fluidvolumen den Spalt vollständig ausfüllt.

4. Bildgebungskatheter nach Anspruch 1, wobei das akustische Kopplungsfluid eine nachfolgende Erzeugung einer vollständigen umlaufenden 360-Grad-Ansicht des umgebenden Gewebes zumindest teilweise basierend auf den akustischen Signalen ermöglicht.

5. Bildgebungskatheter nach Anspruch 1, wobei die Hülle (121) ein Material beinhaltet, das eine hohe mechanische Stabilität umfasst, und gegenüber einer äußeren Umgebung abgedichtet ist, um dadurch das Eindringen von Fluid in ein Inneres der Hülle und in einen Kontakt mit dem einen oder den mehreren akustischen Wandlern zu verhindern.

6. Bildgebungskatheter nach Anspruch 1, wobei der eine oder die mehreren akustischen Wandler an ein Bildgebungsfenster der Hülle ausgerichtet sind.

7. Bildgebungskatheter nach Anspruch 1, wobei die Außenhülse (114) einen oder mehrere Abschnitte entlang einer Länge davon umfasst und jeder Abschnitt ein Material umfasst, das unterschiedliche mechanische Eigenschaften zeigt.

8. Bildgebungskatheter nach Anspruch 7, wobei ein Hauptkörper der Außenhülse (114) ein erstes Material umfasst und ein distales Spitzenelement, das eine distale Spitze der Außenhülse (114) definiert, ein zweites Material umfasst, wobei das erste Material ein elastomeres Material mit relativ hoher Durometerzahl umfasst und das zweite Material ein elastomeres Material mit relativ niedriger Durometerzahl umfasst.

9. Bildgebungskatheter nach Anspruch 1, ferner umfassend eine distale Spitze (116), die an einen distalen Abschnitt der Außenhülse (114) gebunden ist, um dadurch ein Fluid innerhalb der inneren Außenhülse (114) abzudichten.

10. Bildgebungskatheter nach Anspruch 9, wobei die distale Spitze (116) zumindest eines von einem Spülventil und einem Injektionsanschluss umfasst, wobei der Betrieb zumindest eines davon das Entfernen von zumindest Luft aus dem Inneren der Außenhülse (114) erleichtert.

11. Bildgebungskatheter nach Anspruch 9, wobei die Außenhülse (114) ein erstes Material umfasst und die distale Spitze (116) ein zweites Material umfasst, wobei das erste Material ein elastomeres Material mit relativ hoher Durometerzahl umfasst und das zweite Material ein elastomeres Material mit relativ niedriger Durometerzahl umfasst.

12. Bildgebungskatheter nach Anspruch 1, ferner umfassend ein oder mehrere Verbindungselemente, an die zumindest ein proximales Ende der Außenhülse (114) gebunden ist, um dadurch das Fluid innerhalb des Inneren der Außenhülse (114) abzudichten.

13. Bildgebungskatheter nach Anspruch 1, wobei die Drehmomentwelle (138) ein flexibles Rohr zum Übertragen von Drehmomentkräften auf die Wandlereinheit (120) umfasst, um eine Drehung davon zu bewirken.

14. Bildgebungskatheter nach Anspruch 1, ferner umfassend eine Kabelanordnung, die einen oder mehrere Drähte (142) zum Führen von Signalen zu und von der Wandlereinheit (120) für eine nachfolgende Erzeugung einer vollständigen umlaufenden 360-Grad-Ansicht von umgebendem Gewebe zumindest teilweise basierend auf den Signalen beinhaltet.

15. Bildgebungskatheter nach Anspruch 14, wobei die Kabelanordnung zumindest eines von einem verdrillten Paar von Drähten, einem Koaxialkabel und einem flexiblen Leiterplattenkabel umfasst, wobei jedes davon einen proximalen Verbindungsabschnitt von Drähten und einen distalen Verbindungsabschnitt von Drähten umfasst.

## Revendications

1. Cathéter d'imagerie (102) comprenant :
un ensemble d'imagerie (104) pourvu au niveau d'une partie distale du cathéter (102), l'ensemble d'imagerie comprenant une unité de transducteur entièrement rotative (120) comprenant un ou plusieurs transducteurs acoustiques enfermés à l'intérieur d'une coque (121), ladite unité de transducteur (120) étant associée de manière fonctionnelle à un arbre de couple (138) conçu pour provoquer la rotation de l'unité de transducteur (120) lors de son fonctionnement, un manchon externe (114) positionné sur l'ensemble d'imagerie qui entoure l'ensemble d'imagerie de manière à ce qu'un espace soit défini entre l'ensemble d'imagerie (104) et une surface interne du manchon externe (114) de sorte qu'un volume de fluide puisse être reçu à l'intérieur de l'espace, le fluide étant un fluide de couplage acoustique destiné à transporter des signaux acoustiques entre l'unité de transducteur et un tissu environnant, le cathéter d'imagerie étant **caractérisé en ce que**,
l'unité de transducteur (120) est associée de manière fonctionnelle à l'arbre de couple par l'intermédiaire d'un engagement direct entre un ou plusieurs éléments de raccordement femelles (129) d'un mandrin (128) pourvus au niveau d'une extrémité distale de l'arbre de couple et un ou plusieurs éléments de raccordement mâles correspondants (126) pourvus au niveau d'une extrémité proximale de la coque (121) ;
le manchon externe comprenant un matériau sonoluminescent correspondant à l'impédance des signaux acoustiques destinés à être transmis à un tissu environnant ; et
au moins un orifice d'injection et au moins une vanne de purge associés de manière fonctionnelle au manchon externe, ledit orifice d'injection étant conçu pour l'introduction du volume de fluide dans l'espace et la vanne de purge étant conçue pour permettre à au moins un volume de fluide reçu d'être purgé de l'espace.

2. Cathéter d'imagerie selon la revendication 1, un élément parmi l'orifice d'injection et la vanne de purge étant fourni au niveau d'une position proximale par rapport à l'ensemble d'imagerie (104) et l'autre élément étant fourni au niveau d'une position distale par rapport à l'ensemble d'imagerie (104).

3. Cathéter d'imagerie selon la revendication 1, ledit volume de fluide remplissant complètement ledit espace.

4. Cathéter d'imagerie selon la revendication 1, ledit fluide de couplage acoustique permettant la génération ultérieure d'une vue circonférentielle complète à 360 degrés du tissu environnant sur la base, au moins en partie, des signaux acoustiques.

5. Cathéter d'imagerie selon la revendication 1, ladite coque (121) comprenant un matériau comprenant une stabilité mécanique élevée et étant étanche par rapport à un environnement externe pour empêcher ainsi l'entrée de fluide à l'intérieur de la coque et en contact avec lesdits un ou plusieurs transducteurs acoustiques.

6. Cathéter d'imagerie selon la revendication 1, lesdits un ou plusieurs transducteurs acoustiques étant alignés avec une fenêtre d'imagerie de la coque.

7. Cathéter d'imagerie selon la revendication 1, ledit manchon externe (114) comprenant une ou plusieurs parties le long d'une longueur de celui-ci et chaque partie comprenant un matériau présentant des propriétés mécaniques différentes.

8. Cathéter d'imagerie selon la revendication 7, un corps principal du manchon externe (114) comprenant un premier matériau et un élément de pointe distale définissant une pointe distale du manchon externe (114) comprenant un second matériau, ledit premier matériau comprenant un matériau élastomère à dureté relativement élevée et ledit second matériau comprenant un matériau élastomère à dureté relativement faible.

9. Cathéter d'imagerie selon la revendication 1, comprenant en outre une pointe distale (116) liée à une partie distale du manchon externe (114) pour ainsi enfermer hermétiquement un fluide à l'intérieur du manchon externe intérieur (114).

10. Cathéter d'imagerie selon la revendication 9, ladite pointe distale (116) comprenant au moins une vanne de purge et un orifice d'injection, ledit fonctionnement d'au moins un élément parmi ceux-ci facilitant l'élimination d'au moins de l'air de l'intérieur du manchon externe (114).

11. Cathéter d'imagerie selon la revendication 9, ledit manchon externe (114) comprenant un premier matériau et ladite pointe distale (116) comprenant un second matériau, ledit premier matériau comprenant un matériau élastomère à dureté relativement élevée et ledit second matériau comprenant un matériau élastomère à dureté relativement faible.

12. Cathéter d'imagerie selon la revendication 1, comprenant en outre un ou plusieurs éléments de raccordement auxquels au moins une extrémité proximale du manchon externe (114) est liée pour ainsi enfermer hermétiquement le fluide à l'intérieur du manchon externe (114).

13. Cathéter d'imagerie selon la revendication 1, ledit arbre de couple (138) comprenant un tube souple destiné à transmettre des forces de couple sur l'unité de transducteur (120) pour provoquer sa rotation.

14. Cathéter d'imagerie selon la revendication 1, comprenant en outre un ensemble de câbles comprenant un ou plusieurs fils (142) destinés au transport des signaux vers et depuis l'unité de transducteur (120) pour la génération ultérieure d'une vue circonférentielle complète à 360 degrés du tissu environnant sur la base, au moins en partie, des signaux.

15. Cathéter d'imagerie selon la revendication 14, ledit ensemble de câbles comprenant au moins un élément parmi une paire torsadée de fils, un câble coaxial et un câble de circuit imprimé souple, chacun d'eux comprenant une partie d'interconnexion proximale de fils et une partie d'interconnexion distale de fils.
